(19) Europäisches Patentamt
European Patent Office
Office européen des brevets

(11) Publication number: **0 351 684**
**A2**

(12) **EUROPEAN PATENT APPLICATION**

(21) Application number: **89112587.4**

(51) Int. Cl.⁴: **C07K 9/00 , C07K 1/00**

(22) Date of filing: **10.07.89**

(30) Priority: **18.07.88 GB 8817053**

(43) Date of publication of application:
**24.01.90 Bulletin 90/04**

(84) Designated Contracting States:
**AT BE CH DE ES FR GB GR IT LI LU NL SE**

(71) Applicant: **GRUPPO LEPETIT S.P.A.**
**23, Via G. Murat**
**I-20159 Milano(IT)**

(72) Inventor: **Malabarba, Adriano**
**5/A, Via Roma**
**I-20082 Binasco (Milano)(IT)**

(54) **Process for preparing N15, N15-dialkyl derivatives of teicoplanin compounds.**

(57) Improved process for selectively preparing $N^{15}$,$N^{15}$-dialkyl derivatives of teicoplanin compounds which comprises addition of an alkaly metal borohydride in the presence of an excess of a carbonyl compound and a suitable acid.

EP 0 351 684 A2

## PROCESS FOR PREPARING $N^{15}$,$N^{15}$-DIALKYL DERIVATIVES OF TEICOPLANIN COMPOUNDS

The invention relates to a process for preparing $N^{15}$,$N^{15}$-dialkyl derivatives of teicoplanin compounds having the following formula:

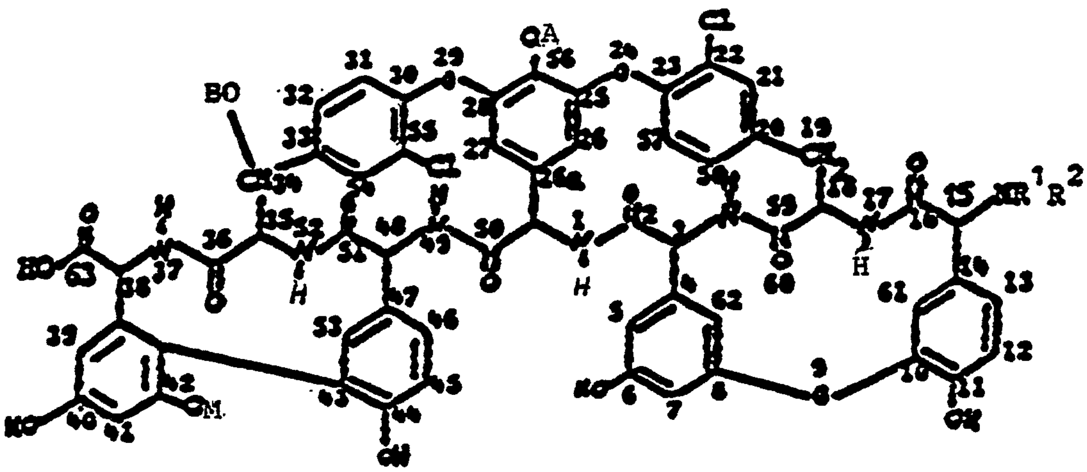

I

wherein

$R^1$ and $R^2$ independently represents ($C_1$-$C_{12}$)alkyl, ($C_4$-$C_7$)cycloalkyl, cyano-($C_1$-$C_3$)alkyl; phenyl-($C_1$-$C_4$)alkyl wherein the phenyl group is optionally substituted in the position ortho, meta and/or para with 1 to 3 groups selected from ($C_1$-$C_4$)alkyl, nitro, halogen, ($C_1$-$C_4$)alkoxy, and phenyl;

A represents hydrogen or -N[($C_9$-$C_{12}$)aliphatic acyl]-beta-D-2-deoxy-2-amino-glucopyranosyl;

B represents hydrogen or N-acetyl-beta-D-2-deoxy-2-amino-glucopyranosyl;

M represents hydrogen or alpha-D-mannopyranosyl; with the proviso that B represents hydrogen only when A and M are simultaneously hydrogen; and the addition salts thereof,

by reductive alkylation of a selected compound of formula II

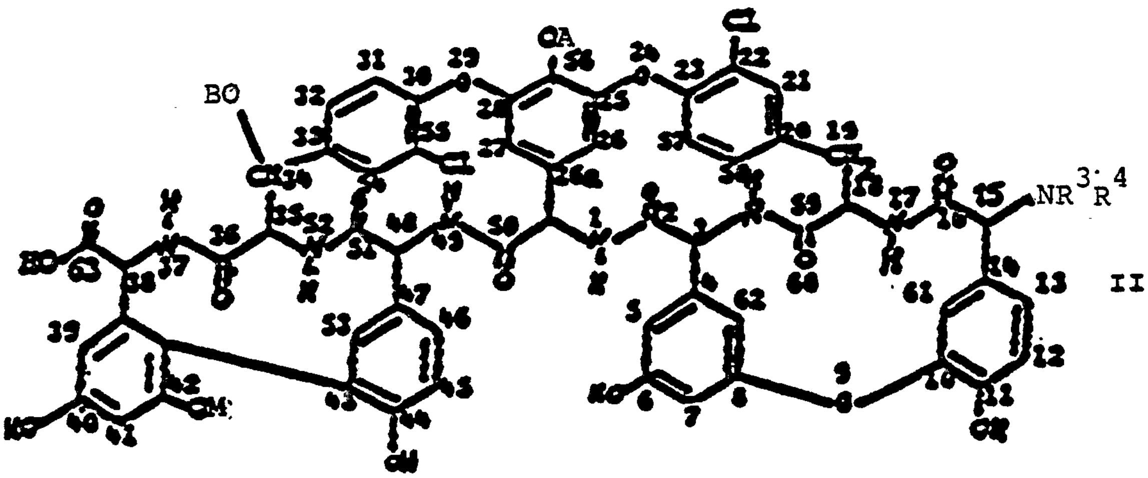

II

wherein $R^3$ is hydrogen and $R^4$ is hydrogen or ($C_1$-$C_{12}$)alkyl, ($C_4$-$C_7$)cycloalkyl, cyano-($C_1$-$C_3$)alkyl; phenyl-($C_1$-$C_4$)alkyl wherein the phenyl group is optionally substituted in the position ortho, meta and/or para with 1 to 3 groups selected from ($C_1$-$C_4$)alkyl, nitro, halogen, ($C_1$-$C_4$)alkoxy, and phenyl as starting material, with a carbonyl compound in the presence of an organic acid of such strength and in such amount that a sample of the obtained mixture diluted with 4 volumes of water shows a pH comprised between 2.5 and 4, and with an alkali metal borohydride in an organic polar solvent.

Teicoplanin is the international non-proprietary name (INN) of the antibiotic substance formerly named teichomycin which is obtained by cultivating the strain Actinoplanes teichomyceticus nov. sp. ATCC 31121

in a culture medium containing assimilable sources of carbon, nitrogen and inorganic salts (see U.S. Patent No. 4,239,751).

According to the procedure described in the above cited patent an antibiotic complex containing Teichomycin $A_1$, $A_2$ and $A_3$ is recovered from the separated fermentation broth by extraction with a suitable water insoluble organic solvent and precipitation from the extracting solvent according to common procedures.

Teichomycin $A_2$, which is the major factor of the isolated antibiotic complex, is then separated from the other factors by means of column chromatography on Sephadex$^R$.

British Patent No. 2121401 discloses that antibiotic Teichomycin $A_2$ actually is a mixture of five closely related co-produced main components.

It is possible to represent teicoplanin $A_2$ (formerly Teichomycin $A_2$) main components 1, 2, 3, 4 and 5 by the above formula I wherein $R^1$ and $R^2$ are hydrogen, A represents -N[($C_{10}$-$C_{11}$)aliphatic acyl]-beta-D-2-deoxy--2-amino-glucopyranosyl, B represents N-acetyl-beta-D-2--deoxy-2-amino-glucopyranosyl, M represents alpha-D-mannopyranosyl.

More particularly, in teicoplanin $A_2$ component 1, the [($C_{10}$-$C_{11}$)-aliphatic acyl] substituent represents Z-4-decenoyl, in teicoplanin $A_2$ component 2 represents 8-methyl-nonanoyl, in teicoplanin $A_2$ component 3 represents decanoyl, in teicoplanin $A_2$ component 4 represents 8-methyldecanoyl, in teicoplanin $A_2$ component 5 represents 9-methyldecanoyl.

European Patent Application Publication No. 306645 describes production of teicoplanin compounds where the aliphatic acyl group of the beta-D-2-deoxy-2-amino-glycopyranosyl moiety is a n-nonanoyl (compound B or RS3) or a 6-methyloctanoyl (compound A or RS4) group.

In the paper entitled: "Isolation by HPLC and structural determination of minor components of teicoplanin" given by Zanol et al., at the 17th International Symposium on chromatography, Wien, September 25-30, 1988, other two teicoplanin compounds (RS1 and RS2) are described.

Said compounds are characterized in that the aliphatic acyl moieties of the beta-D-2-deoxy-2-amino-glucopyranosyl moiety are respectively methyl-undecanoyl and dodecanoyl.

All the sugar moieties, when present, are linked to the teicoplanin nucleus through O-glycosidic bonds.

In addition, it has been found that it is possible to transform teicoplanin, a pure factor thereof or a mixture of any of said factors in any proportion, into unitary antibiotic products by means of selective hydrolysis of one or two sugar moieties.

They are named antibiotic L 17054 and antibiotic L 17046 and are described in European Patent Application Publication No. 119575 and European Patent Application Publication No. 119574, respectively.

Preferred hydrolysis conditions for the production of antibiotic L 17054 are: 0.5 N hydrochloric acid at a temperature between 70 °C and 90 °C and for a time which is generally between 15 and 90 min.

Antibiotic L 17054 is represented by the above formula I wherein $R^1$, $R^2$ and A represent hydrogen, B represents N-acetyl-beta-D-2-deoxy-2-amino-glucopyranosyl, M represents alpha-D-mannopyranosyl wherein the sugar moieties are linked to the peptidic nucleus through an O-glycosidic bond.

Preferred hydrolysis conditions for the preparation of antibiotic L 17046 are: 1-3 N hydrochloric acid, at a temperature between 50 ° and 90 °C and for a time which is generally between 30 and 60 min.

Antibiotic L 17046 is represented by the above formula I wherein $R^1$, $R^2$, A and M represent hydrogen atoms, and B is N-acetyl-beta-D-2-deoxy-2-aminoglucopyranosyl wherein the sugar moiety is linked to the peptidic nucleus through an O-glycosidic bond.

European Patent Application publication No. 301247 describes de-mannosyl teicoplanin derivatives, i.e. compounds of the formula I above wherein A and B are different from hydrogen and M is hydrogen.

The complete selective cleavage of all the sugar moieties of the teicoplanin compounds gives an aglycone molecule which is called antibiotic L 17392, or deglucoteicoplanin, and is represented by the above formula I wherein $R^1$, $R^2$, A, B, and M each individually represents a hydrogen atom. This selective hydrolysis process is described in European Patent Application Publication No. 146053.

A substance having the same structural formula is disclosed in European Patent Application Publication No. 0090578 and is named antibiotic A 41030 factor B.

This substance is obtained by means of a microbiological process which involves the fermentation of the strain Streptomyces virginiae NRRL 12525 or Streptomyces virginiae NRRL 15156 in a suitable medium, the isolation, purification and separation into its components of antibiotic A 41030, an antibiotic complex of at least seven factors, antibiotic A 41030 factor B, included.

All the above named compounds, i.e. teicoplanin, teicoplanin $A_2$ complex, teicoplanin $A_2$ component 1, teicoplanin $A_2$ component 2, teicoplanin $A_2$ component 3, teicoplanin $A_2$ component 4, teicoplanin $A_2$ component 5, antibiotic L 17054, antibiotic L 17046, antibiotic L 17392, compound A or RS3, compound B or RS4, RS1, RS2, the de-mannosyl teicoplanin derivatives of European Patent Application Publication No.

301247 and any mixture thereof in any proportion, are suitable starting materials for the preparation of the alkyl derivatives of the invention.

In the present specification "teicoplanin compound" or "teicoplanin starting material" is used to indicate any one of the above starting materials, i.e. teicoplanin as obtained according to U.S. patent 4,239,751, any further purification thereof, teicoplanin $A_2$ complex, a compound of the above formula I wherein $R^1$ and $R^2$ are hydrogen, A represents hydrogen or -N[($C_9$-$C_{12}$)aliphatic acyl]-beta-D-2-deoxy-2-amino-glucopyranosyl, B represent hydrogen or N-acetyl-beta-D-2-deoxy-2-amino-glucopyranosyl, M represents hydrogen or alpha-D-mannopyranosyl, with the proviso that B may represent hydrogen only when A and M are simultaneously hydrogen, a salt thereof, or a mixture thereof in any proportion.

As used herein the term "alkyl", either alone or in combination with other substituents, includes both straight and branched hydrocarbon groups; more particularly, "($C_1$-$C_6$)alkyl" represents a straight or branched aliphatic hydrocarbon chain of 1 to 6 carbon atoms such as methyl, ethyl, propyl, 1-methylethyl, butyl, 1-methylpropyl, 1,1-dimethylethyl, pentyl, 1-methylbutyl, 2-methylbutyl, 1-hexyl, 2-hexyl, 3-hexyl, 3,3-dimethyl-1-butyl, 4-methyl-1-pentyl and 3-methyl-1-pentyl; likewise, "($C_1$-$C_4$)alkyl" represents a straight or branched hydrocarbon chain of 1 to 4 carbon atoms like those exemplified above.

The term "halogen" represents an halogen atom selected from chlorine, bromine and iodine.

The compounds of the invention contain acid and basic functions and can form, in addition to "internal salts", acid and base addition salts according to conventional procedures.

For the scope of the present description and claims the "internal salts" are encompassed by the definition of "non-salt" or "non-addition salt" form.

Preferred addition salts are the pharmaceutically acceptable acid and/or base addition salts.

With the term "pharmaceutically acceptable acid and/or base addition salts" are intended those salts with acids and/or bases which from biological, manufacturing and formulation standpoint are compatible with the pharmaceutical practice as well as with the use in the animal growth promotion.

Representative and suitable acid addition salts of the compounds of formula I include those salts formed by standard reaction with both organic and inorganic acids such as, for example, hydrochloric, hydrobromic, sulfuric, phosphoric, acetic, trifluoroacetic, trichloroacetic, succinic, citric, ascorbic, lactic, maleic, fumaric, palmitic, cholic, pamoic, mucic, glutamic, camphoric, glutaric, glycolic, aspartic, phthalic, tartaric, lauric, stearic, salicylic, methanesulfonic, benzenesulfonic, sorbic, picric, benzoic, cinnamic and the like acids and salts with aminoacids like lysine, arginine, asparagine, glutamine, hystidine, serine, methionine, leucine and the like.

Representative examples of these bases are: alkali metal or alkaline-earth metal hydroxide such sodium, potassium, and calcium hydroxide; and organic aliphatic, alicyclic or aromatic amines such as methylamine, dimethylamine, trimethylamine, and picoline.

A preferred group of compounds prepared with the process of the present invention is represented by those compounds of formula I wherein $R^1$ and $R^2$ independently represents ($C_1$-$C_{12}$)alkyl, ($C_4$-$C_6$)cycloalkyl, cyano-($C_1$-$C_2$)alkyl, phenyl($C_1$-$C_3$)alkyl wherein the phenyl moiety is optionally substituted with 1 or 2 substituents selected from methyl, nitro, bromo, chloro, iodo or methoxy.

Another preferred group of compounds prepared by means of the process of the present invention is represented by those compounds of formula I wherein $R^1$ and $R^2$ independently, or both $R^1$ and $R^2$ represent ($C_1$-$C_4$)alkyl and A, B, and M are as defined.

A further preferred group of compounds which can be prepared with the process of the invention is represented by those compounds of formula I wherein $R^1$ and $R^2$ independently represents ($C_1$-$C_4$)alkyl and A, B, and M represent the sugar moieties, defined above or A, B, and M, represent hydrogen atoms.

Preferred compounds prepared by the process of the invention are those compounds of formula I wherein A, B and M represent the sugar moieties defined above or A, B and M represent hydrogen atoms. Further preferred compounds prepared by this process are those compounds of formula I wherein A, B and M represent the sugar moieties defined above and the N[($C_9$-$C_{12}$)aliphatic acyl]-substituent represents 8-methylnonanoyl.

Other preferred compounds which can be prepared with the process of the present invention are:

- a compound of formula I wherein $R^1$ and $R^2$ are methyl.
- a compound of formula I wherein $R^1$ and $R^2$ are ethyl.
- a compound of formula I wherein $R^1$ (or $R^2$) is methyl and $R^2$ (or $R^1$) is ethyl.

The transformation of the free amino or non-salt compounds of the invention into the corresponding addition salts, and the reverse, i.e. the transformation of an addition salt of a compound of the invention into the non-salt or free amino form, are within the ordinary technical skill and are encompassed by the present invention.

For instance, a compound of formula I can be transformed into the corresponding acid or base addition-salt by dissolving the non-salt form in an aqueous solvent and adding a slight molar excess of the selected acid or base.

The resulting solution or suspension is then lyophilized to recover the desired salt. Instead of lyophilizing, in some instances, it is possible to recover the final salt by extraction with an organic solvent, concentration to a small volume of the separated organic phase and precipitation by adding a non-solvent.

In case the final salt is insoluble in an organic solvent where the non-salt form is soluble it is recovered by filtration from the organic solution of the non-salt form after addition of the stoichiometric amount or a slight molar excess of the selected acid or base.

The non-salt form can be prepared from a corresponding acid or base salt dissolved in an aqueous solvent which is then neutralized to free the non-salt form.

This is then recovered for instance by extraction with an organic solvent or is transformed into another base or acid addition salt by adding the selected acid or base and working up as above.

When following the neutralization desalting is necessary, a common desalting procedure may be employed.

For example, column chromatography on controlled pore polydextrane resins (such as Sephadex LH 20) or silanized silica gel may be conveniently used.

After eluting the undesired salts with an aqueous solution, the desired product is eluted by means of linear gradient or step-gradient of a mixture of water and a polar or apolar organic solvent, such as acetonitrile/water from 50:50 to about 100% acetonitrile.

As is known in the art, the salt formation either with pharmaceutically acceptable acids (bases) or non-pharmaceutically acceptable acids (bases) may be used as a convenient purification technique.

After formation and isolation, the salt form of a compound of formula I can be transformed into the corresponding non-salt or into a pharmaceutically acceptable salt.

In some instances the acid addition salt of a compound of formula I is more soluble in water and hydrophilic solvents and has an increased chemical stability.

However, in view of the similarity of the properties of the compounds of formula I and their salts, what is said in the present application when dealing with the biological activities of the compounds of formula I applies also to their pharmaceutically acceptable salts, and viceversa.

The compounds prepared by means of the process of the present invention are useful as semi-synthetic antibacterial agents mainly active against gram positive bacteria or as growth promoters.

An object of the present invention is a process for selectively obtaining with good yields symmetrical or unsymmetrical $N^{15}$,$N^{15}$-di-alkyl derivatives of teicoplanin compounds.

As known in fact, when mixed hydrides or suitable reducing agents selectively reduce a Schiff base in the presence of an excess of a carbonyl compound, usually the final mixture contains both mono-alkyl derivatives and di-alkyl ones. In said mixture, di-alkyl derivatives can be obtained in good yields but it is very troublesome to separate them from the mono-alkyl components.

We have found a method for preparing di-alkyl derivatives from teicoplanin compounds which has the advantage of having high selectivity together with fast conversion rate, very high yields and absence of side-reactions and by-products. Moreover, it allows a simple and fast "one pot" reaction process leading to the desired compounds, possibly after ordinary purification steps such as desalting, thus avoiding sophisticated and expensive purification steps, such as preparative HPLC.

Said process comprises adding to a suspension of a teicoplanin compound or a compound of formula II wherein $R^3$ is H and $R^4$ is H or $(C_1$-$C_{12})$alkyl, $(C_4$-$C_7)$cycloalkyl, cyano-$(C_1$-$C_3)$alkyl; phenyl-$(C_1$-$C_4)$alkyl wherein the phenyl group is optionally substituted in the position ortho, meta and/or para with 1 to 3 groups selected from $(C_1$-$C_4)$alkyl, nitro, bromo, chloro, iodo, $(C_1$-$C_4)$alkoxy, and phenyl, in an organic polar protic solvent a molar excess of a carbonyl compound having the same carbon skeleton of the substituents $R^1$ and $R^2$ above and whose oxo group is on the carbon atom which is to link the $N^{15}$-amino group of the teicoplanin moiety, said molar excess being of at least 30 times with respect to the teicoplanin starting material, in the presence of an organic acid of such strength and in such amount that a sample of the obtained mixture diluted with four volumes of water shows a pH between 2.5 and 4, said acid being further characterized in that, under the reaction conditions, it cannot yield a reduction product which is a competitor of the carbonyl compound reactant in the reaction with the $N^{15}$ amino group of the teicoplanin moiety, and then reacting the resulting mixture with a metal alkali borohydride at a temperature between 0° C and 60° C.

Among the organic polar protic solvents, alkyl alcohols having from 1 to 4 carbon atoms are preferred.

Examples of alcohols which can be used are methanol, ethanol, propanol and butanol. In particular, methanol is preferred.

Sometimes in particular cases, a small amount of a polar co-solvent can be added to completely dissolve teicoplanin starting material during the course of the reaction, e.g. N,N-dimethylformamide, 1,3-dimethyl-3,4,5,6-tetrahydro-2(1H)-pyramidone (DMPU), dimethylsulfoxide.

As the reducing agent, alkali metal borohydrides are used, among which sodium borohydride is the preferred one, but also potassium and sodium cyano-borohydrides can be conveniently used.

The temperature can be preferably maintained between 30 °C and 40 °C.

The molar excess of the reactants with respect to teicoplanin ranges preferably from 30 to 300 times for aldehydes or ketones.

The organic acid which is used in the present invention is characterized by having such a strength to show a pH between 2.5 and 4 when a sample of the reaction mixture is added to 4 volumes of water before adding the reducing agent. Furthermore, said acid must not negatively interfere in the reaction course. It means that it preferably has not to be reduced by the reducing agent employed ($NaBH_4$ and the like) or, if a reaction occurs, the product of this reaction is not a competitor with the carbonyl compound choosen as starting material.

The term "competitor" as used in specification and claims means any group which can be linked to an amino group with a bond like a "Schiff base" and subsequently reduced to an alkyl moiety, being said group different from the carbonyl compound used as starting material. Thus, in a preferred embodiment of the present invention when an aldehyde is used as the carbonyl compound the suitable acid to be preferably used is the carboxylic acid corresponding to said aldehyde; for example, formic acid with formaldehyde, acetic acid with acetaldehyde and so on. In the case that a keto compound is used as the starting material, a preferred acid can be choosen among a ($C_1$-$C_6$)alkyl sulfonic acid and a ($C_6$-$C_{10}$)aryl sulfonic acid such as for example paratoluensulfonic acid, methanesulfonic acid and the like. The amount of acid used depends on several factors such as the teicoplanin starting material and the carbonyl compound but usually the desired pH is obtained when a molar excess of acid is used from 5 to 20 times with respect to teicoplanin.

The mono alkyl derivatives, which can be used as starting material for the process of the present invention can be prepared according to a variety of alkylation procedures.

For example, they can be prepared by reacting a teicoplanin compound with an alkyl halide in the presence of a base according to the European Patent Application Publication No. 276740 in the name of Applicant.

Said mono alkyl derivatives can be also prepared by reductive alkylation as described in the copending European Patent Application claiming the priority of UK Application Ser. No. 8817052.7 filed on July 18, 1988 in the name of Applicant.

A preferred procedure for preparing said mono-alkyl starting material is represented by an alkylation with an alkyl halide $R^1X$ or $R^2X$ wherein the alkyl portion is represented by an "alkyl" group which corresponds to the desired meaning of $R^1$ or $R^2$ in the final product and the halide is preferably a chloride, bromide or iodide and most preferably bromide or chloride.

The reaction occurs at about room temperature in the presence of an organic or inorganic base of medium strength such as triethylamine, trimethylamine, tri-butylamine, sodium or potassium bicarbonate and the like.

Preferably, the reaction medium includes also an organic solvent capable of at least partially solubilizing the starting materials such as organic amides, alkyl ethers, ethers of glycols and polyols, phosphoramides, sulfoxides and aromatic compounds e.g., dimethylformamide, dimethoxyethane, hexamethylphosphoramide, dimethylsulfoxide, benzene, toluene and mixtures thereof.

This solvent should have a low water content (generally below about 5%).

The alkyl halide and the starting teicoplanin derivative are preferably employed in about equimolecular proportion or in a slight molar excess of the alkyl halide so that the mono-alkyl derivative is obtained in good yields.

As already said, the reaction occurs at about room temperature so that external heating is not in general necessary, even if the reaction can be carried out at temperatures of 40-70 °C.

The carbonyl compound used as starting reactant has the same carbon skeleton of the substituents $R^1$ and/or $R^2$ and has the oxo group on the carbon atom which is desired to link the $N^{15}$-teicoplanin moiety.

A skilled technician is able to easily recognize the useful carbonyl compound which has to be used in order to obtain the desired $N^{15}$, $N^{15}$ dialkyl derivative of teicoplanin. For instance, if the desired $N^{15}$,$N^{15}$ dialkyl substituents $R^1$ and $R^2$ are the same and represent two

- CH — $CH_2$-$CH_3$ groups,
  |
  $CH_3$

the corresponding oxo alkyl compound used as the starting material is methyl ethyl ketone,

$CH_3$- C -$C_2H_5$
  ||
  O

If the alkyl groups linked to the $N^{15}$ atom of teicoplanin are different it is necessary to prepare a $N^{15}$ monoalkyl derivative according to one of the methods described above and then to employ said derivative as starting material.

Thus, for example if $-\underset{\displaystyle CH_3}{\underset{|}{C}}HCH_2CH_3$ and $-\underset{\displaystyle CH_3}{\underset{|}{C}}HCH_3$ are the desired substituents for $R^1$ and $R^2$ respectively, the teicoplanin compound is reacted with $CH_3-\underset{\displaystyle Br}{\underset{|}{C}}H-CH_3$ in the presence of a base, and then the $N^{15}$ monoalkyl product so obtained is reacted with $CH_3-\underset{\displaystyle O}{\underset{||}{C}}-CH_2CH_3$ in the presence of a suitable acid by a reductive agent according to the present invention.

The sugar moiety of an $N^{15}$,$N^{15}$-di-alkyl compound of formula I may be selectively removed to transform it into a different compound having the same general formula.

For example, an $N^{15}$,$N^{15}$-di-alkyl compound of formula I wherein A, B, and M represent a sugar moiety as above defined can be transformed into the corresponding compound wherein B and M are as above and A is hydrogen by means of controlled acid hydrolysis in a strong concentrated aqueous organic acid.

The concentrated organic acid in this case is preferably aqueous trifluoroacetic acid at a concentration between 75% and 95%, and the reaction temperature is preferably between 10° and 50° C.

The preferred hydrolysis conditions are represented by about 90% trifluoroacetic acid at room temperature.

The reaction time varies depending on the other specific reaction parameters but, in any case, the reaction may be monitored by TLC or preferably HPLC techniques.

An analogous selective hydrolysis is reported in European Patent Application Publication No. 146822.

Similarly, a $N^{15}$,$N^{15}$-di-alkyl compound of formula I wherein A, B, and M represent a sugar moiety as above defined or A represents hydrogen and B and M represent sugar moieties as above defined can be transformed into the corresponding alkyl compounds of formula I wherein A and M represent hydrogen and B represent a sugar moiety as defined by means of a selective hydrolysis with a strong acid in the presence of a polar aprotic solvent selected from ethers, ketones, and mixture thereof which are liquid at room temperature.

Preferred hydrolysis conditions are in this case represented by the use of a concentrated mineral acid in the presence of an ether such as dimethoxyethane at room temperature.

The reaction course may be monitored by TLC or preferably HPLC. An analogous selective hydrolysis is reported in European Patent Application Publication No. 175100.

According to another embodiment of the present invention, a $N^{15}$,$N^{15}$-di-alkyl compound of formula I wherein A, B and M represent sugar moieties as defined above, a $N^{15}$,$N^{15}$-di-alkyl compound of formula I wherein A represents hydrogen and B and M represent the above defined sugar moieties, or a $N^{15}$,$N^{15}$-di-alkyl compound of formula I wherein A and M represent hydrogen, and B represents a sugar moiety as above defined, may be transformed into the corresponding alkyl compound of formula I wherein A, B and M represent hydrogen atoms by means of a selective hydrolysis in an organic protic solvent selected from aliphatic acids and alpha-halogenated aliphatic acids which at the reaction temperature are liquids, aliphatic and cycloaliphatic alkanols which at the reaction temperature are liquids slightly mixable with water, phenyl-substituted lower alkanols wherein the phenyl moiety may optionally carry $(C_1-C_4)$alkyl, $(C_1-C_4)$alkoxy or halo rests which at the reaction temperature are liquids slightly mixable with water, and beta-poly-halogenated lower alkanols, which at the reaction temperature are liquids; in the presence of a strong acid, compatible with the solvent, selected from strong mineral acids, strong organic acids and strong acid cation exchanger resins in the hydrogen form and at a temperature between 20° C and 100° C.

In this case, the preferred hydrolysis conditions are represented by the use of a mineral acid, such as hydrochloric acid, in an haloalkanol such as trifluoroethanol, at a temperature between 65° C and 85° C.

Analogous selective hydrolysis conditions on a similar substrate are described in European Patent Application Publication No. 146053.

Surprisingly, carrying out the alkylation reduction in the presence of an organic acid having a pH comprised between 2.5 and 4 (when measured as described above), it is possible to increase in a relevant way the yield of the final product.

For example, by starting from a teicoplanin compound as in formula I wherein A is $-N[(C_9-C_{12})$ aliphatic

acyl]-beta-D-2-deoxy-2-amino-glucopyranosyl, B is N-acetyl-beta-D-2-deoxy-2-amino-glucopyranosyl, M is alpha-D-mannopyranosyl, $R^1$ is H and $R^2$ is $C_2H_5$, by using formaldehyde as carbonyl compound and $NaBH_4$ as metal alkali borohydride, the yield of the $N^{15}$,$N^{15}$ methyl ethyl derivative is about 60% (see example 1I, page 43, European Patent Application Publication No. 276740).)

By repeating the same reaction but in the presence of a suitable amount of formic acid before adding $NaBH_4$, the yield of the $N^{15}$,$N^{15}$ methyl ethyl derivative is about 93% as described in Example 2 below.

In the following table (Table I) the structure formulas of representative compounds of the invention are reported.

TABLE I

| COMPOUND | A | B | M | $R^1$ | $R^2$ | YIELD % |
|---|---|---|---|---|---|---|
| 1 | $-GNHCOR_{(1-5)}$ | $-GNHCOCH_3$ | -M | $CH_3$ | $CH_3$ | 95% |
| 2 | do | do | do | $CH_3$ | $C_2H_5$ | * |
| 3 | do | do | do | $C_2H_5$ | $C_2H_5$ | 91% |

* Starting from mono-alkyl compound wherein $R^1$ is H and $R^2$ is $CH_3$: 91%
Starting from mono-alkyl compound wherein $R^1$ is H and $R^2$ is $C_2H_5$: 93%

$-GNHCOR_{(1-5)}$ represents -N[($C_{10}$-$C_{11}$)aliphatic acyl]-beta-D-2-deoxy-2-amino-glucopyranosyl

continued... TABLE I

| COMPOUND | A | B | M | $R^1$ | $R^2$ | YIELD % |
|---|---|---|---|---|---|---|
| 4 | H | H | H | $CH_3$ | $CH_3$ | 91% |
| 5 | do | do | do | $C_2H_5$ | $C_2H_5$ | 92% |
| 6 | do | do | do | $CH_3$ | $C_2H_5$ | (*) |

* Starting from mono-alkyl compound wherein $R^1$ is H and $R^2$ is $CH_3$: 89%
Starting from mono-alkyl compound wherein $R^1$ is H and $R^2$ is $C_2H_5$: 98%

continued... TABLE I

| COMPOUND | A | B | M | $R^1$ | $R^2$ | YIELD % |
|---|---|---|---|---|---|---|
| 7 | $-GNHCOR_{(2)}$ | $-GNHCOCH_3$ | -M | $CH_3$ | $CH_3$ | - |
| 8 | do | do | do | $CH_3$ | $C_2H_5$ | - |
| 9 | do | do | do | $C_2H_5$ | $C_2H_5$ | - |

-GNHCOR(2) represents -N[8-methyl-nonanoyl]-beta-D-2-deoxy-2-aminoglucopyranosyl

HPLC Analysis

The following table (Table II) reports the $t_R$ of representative examples of the compounds that can be prepared according to the process of the invention.

The assays were run with a HPLC Hewlett - Packard 1084 apparatus equipped with UV detector (254 nm).

Columns:

Stainless steel Precolumn Hibar Lichro CART packed with Lichrosorb RP-8 Merck (silanized Silica gel, 5 micrometer) followed by a Hibar LichrocART column (25 cm) pre-packet with Lichrosorb RP-8 (7 micrometer)
Flow rate : 1.5 ml/min
Injection volume : 30 microliter

Eluents:

Solution A: $CH_3CN/NaH_2PO_4$ (0.02 M), 5/95 (v/v)
Solution B: $CH_3CN/NaH_2PO_4$ (0.02 M), 75/25 (v/v)

| Gradient profile: | | | | | | |
|---|---|---|---|---|---|---|
| minute:<br>% B in A | 0<br>8 | 40<br>40 | 45<br>60 | 48<br>65 | 55<br>8 | 56<br>- |

TABLE II

| (HPLC Analysis) | | |
|---|---|---|
| No. COMPOUND | $t_R$ | $t_R/t_R$ L 17392 |
| 1 | 28.13-29.03*-29.76 | 1.88 |
| | 32.54-33.12* | 2.15 |
| 4 | 18.11 | 1.20 |
| Deglucoteicoplanin (standard) | 15.30 | 1.00 |

* Peak utilized for the ratio calculation.

The following table (TABLE III) reports the acid-base titration of a representative compound that can be prepared according to the process of the invention. The assay is carried out in Methylcellosolve $^R/H_2O$, 4/1 (v/v). A sample (10 micromole) is dissolved in about 20 ml, then 0.01 N HCl (2 ml) is added and the mixture is titrated with 0.01 N NaOH in the same solvent mixture.

TABLE III

Acid base titration

| COMPOUND | Salt Form | $pK_1$ | $pK_2$ |
|---|---|---|---|
| 1 | HCl | 4.8 | 6.9 |

The I.R. most significant bands ($cm^{-1}$) of some compounds that can be prepared according to the process of the invention recorded with a Perkin-Elmer 580 spectrometer in nujol mull are reported below (o.b. = overlapped band):

TABLE IV

IR Data ($cm^{-1}$, nujol)

| COMPOUND | $\nu$ NH<br>Glycosidic and phenolic OH | $\nu$ C=O (COOH)<br>(amide I) | $\delta$NH<br>(amide II) | Glucoside<br>$\delta$OH, $\nu$C-O | Phenolic<br>$\nu$C-O |
|---|---|---|---|---|---|
| 1 | 3700-3100 | 1725 (COOH)<br>1650 (amide I) | 1510 | 1250-1190<br>1100-930 | o.b. |
| 2 | 3700-3100 | 1655 (amide I) | 1505 | 1250-1190<br>1100-930 | o.b. |

Continued... TABLE IV

| COMPOUND | ν NH Glycosidic and phenolic OH | ν C=0 (COOH) (amide I) | δ NH (amide II) | Glucoside δOH, νC-O | Phenolic ν C-O |
|---|---|---|---|---|---|
| 3 | 3700-3100 | (COOH) 1655 (amide I) | 1510 | 1250-1190 1100-930 | o.b. |
| 5 | 3700-3100 | 1725 (COOH) 1650 (amide I) | 1515 | | 1060, 1010 |
| 6 | 3700-3100 | 1645 (amide I) | 1510 | | 1060, 1010 |

## TABLE V

UV-Vis data of some representative compounds that can be prepared according to the process of the invention recorded with a Unicam SP 500 spectrophotometer are reported below (Lambda max, nm):

| Solvent | Compound No. 1 | Compound No. 2 | Compound No. 3 |
|---|---|---|---|
| Methanol | 280 | | |
| 0.1N HCl | 280 | 279 | 280 |
| Phosphate buffer pH 7.4 | 279-280 | 279 | 279 |
| Phosphate buffer pH 9.0 | 280 | | |
| 0.1N KOH | 299 | 298 | 298 |

Continued... TABLE V

| Solvent | Compound No. 5 | Compound No. 6 |
|---|---|---|
| Methanol | | |
| 0.1N HCl | 278 | 279 |
| Phosphate buffer pH 7.4 | 278 | 279 |
| Phosphate buffer pH 9.0 | | |
| 0.1N KOH | 297 | 297 |

Table VI reports $^1H$-NMR data obtained at 250 MHz with a Bruker AM-250 Spectrometer in DMSO-$D_6$ at 20°C, at a sample concentration of 20 mg/ml (internal standard : TMS, delta = 0.00 ppm).

TABLE VI

| COMPOUND | |
|---|---|
| 1 | 0.83, 1.13-1.22, 1.44, 2.03 (acyl chain); 1.87 (acetylglucosamine); 2.49 ($N^+-CH_3$); 4.09-5.70 (peptidic CH's); 6.20-8.60 (aromatic CH's and peptidic NH's) |
| 2 | 2.25 ($N-CH_3$); 1.06, 3.06 ($N-C_2H_5$); 0.83, 1.39, 2.03 (acyl chain) 4.08-5.67 (peptidic CH's); 6.25-8.42 (aromatic protons a peptidic NH's) |
| 3 | 0.83, 1.39, 2.02 (acyl chain); 1.84 (acetylglucosamin); 1.04, 3.07 ($N-C_2H_5$); 3.47 (mannose); 4.11-5.71 (peptidic CH's); 6.29-8.38 aromatic protons a peptidic NH's) |

Continued... TABLE VI

| COMPOUND | |
|---|---|
| 4 | 0.89, 1.45, 2.49 (N-propyl chain); 4.12-5.66 (peptidic CH's); 5.50 ($C-_{27}$ H); 5.10 ($C-_{26}$ H); 6.25-8.45 (aromatic CH's and peptidic NH's) |
| 5 | 1.21, 3.14 ($N-C_2H_5$); 4.08-5.58 (peptidic CH's); 5.08 ($C_{26}$-H); 5.45 ($C_{27}$-H); 6.23-8.51 (aromatic protons and peptidic NH's) |
| 6 | 2.28 ($N-CH_3$); 0.96, 2.52, ($N-C_2H_5$); 4.04-5.66 (peptidic CH's); 5.09 ($C_{26}$-H); 5.49 ($C_{27}$-H); 6.24-8.37 (aromatic protons a peptidic NH's) |

The antibacterial activity of the compounds prepared by means of the process of the present invention can be demonstrated in vitro by means of standard agar-dilution tests.

Isosensitest broth (Oxoid) and Todd-Hewitt broth (Difco) are used for growing staphylococci and streptococci, respectively. Broth cultures are diluted so that the final inoculum is about $10^4$ colony forming units/ml (CFU/ml). Minimal inhibitory concentration (MIC) is considered as the lowest concentration which shows no visible growth after 18-24 h incubation at 37° C. The results of the in vitro antibacterial testing of representative compounds of formula I are summarized in Table VII.

TABLE VII

*In vitro* (MIC microgram/ml)

| Test organism | Compound No. 1 | Compound No. 2 | Compound No. 3 |
|---|---|---|---|
| *S. haemolyticus* L 602 | n.d. | 8 | 4 |
| *S. aureus* Tour | 0.25 | 0.25 | 0.25 |
| *S. aureus* Tour + 30% bovine serum | 0.5 | 1 | 2 |
| *S. epidermidis* ATCC 12228 | 0.25 | 0.5 | 0.12 |
| *S. pyogenes* C203 | 0.06 | 0.06 | 0.06 |
| *S. pneumoniae* UC41 | 0.06 | 0.12 | 0.12 |
| *S. faecalis* ATCC 7080 | 0.12 | 0.12 | 0.25 |
| *E. coli* SKF 12140 | >128 | >128 | >128 |
| *P. vulgaris* X19H ATCC 881 | >128 | >128 | >128 |
| *P. aeruginosa* ISM | >128 | >128 | >128 |

Continued... TABLE VII

| Test organism | Compound No. 4 | Compound No. 5 | Compound No. 6 |
|---|---|---|---|
| *S. haemolyticus* L 602 | n.d. | 0.25 | 0.25 |
| *S. aureus* Tour | 0.12 | 0.12 | 0.12 |
| *S. aureus* Tour + 30% bovine serum | 0.25 | 0.5 | 0.5 |
| *S. epidermidis* ATCC 12228 | 0.03 | 0.06 | 0.06 |
| *S. pyogenes* C203 | 0.12 | 0.12 | 0.06 |
| *S. pneumoniae* UC41 | 0.12 | 0.12 | 0.12 |
| *S. faecalis* ATCC 7080 | 0.25 | 0.12 | 0.12 |
| *E. coli* SKF 12140 | >128 | >128 | >128 |
| *P. vulgaris* X19H ATCC 881 | >128 | >128 | >128 |
| *P. aeruginosa* ISM | >128 | >128 | >128 |

The $ED_{50}$ values (mg/Kg) of representative compounds prepared by the process of the invention in vivo tests in mice experimentally infected with S. pyogenes L 49 according to the procedure described by V. Arioli et al., Journal of Antibiotics 29, 511 (1976) are in the range of 0.17-5.0 mg/kg s.c., and mainly between 0.17 and 1.25 mg/Kg s.c., and in the range 170-300 mg/Kg or more per os.

In view of the above reported antimicrobial activity, the compounds prepared according to the present invention can effectively be employed as the active ingredients of antimicrobial preparations used in human and veterinary medicine for the prevention and treatment of infectious diseases caused by pathogenic bacteria which are susceptible to said active ingredients.

In such treatments, these compounds may be employed as such or in the form of mixtures in any proportion, or pharmaceutical compositions in admixture with the known pharmaceutically acceptable carriers or excipients.

The compounds prepared by the process of the present invention can be administered orally, topically or parenterally wherein however, the parenteral administration is preferred.

Depending on the route of administration, these compounds can be formulated into various dosage forms.

Preparations for oral administration may be in the form of capsules, tablets, liquid solutions or suspensions which can be prepared according to known per se techniques.

The compounds prepared according to the process of the invention are generally effective at a dosage comprised between about 0.5 and about 30 mg of active ingredient per Kg of body weight, preferably divided in 2 to 4 administrations per day. Particularly desirable compositions are those prepared in the form of dosage units containing from about 20 to about 300 mg per unit.

Besides their activity as medicaments, the compounds prepared according to the process of the present invention can be used as animal growth promoters preferably in admixture with conventional carrier or feed.

The following examples further illustrate the invention and must not be construed as limiting it.

Example 1

Preparation of Compound 1

To a stirred suspension of 2 g (about 1 mmol) of teicoplanin $A_2$ complex in 100 ml of methanol, 4 ml (about 53 mmol) of 40% (by weight) chilly aqueous formaldehyde and 0.4 ml (about 7 mmol) of anhydrous formic acid are added. The clear solution which forms is warmed to 30°C and 0.54 g (about 15 mmol) of sodium borohydride is added portionwise, while maintaining the reaction temperature between 35°C and 40°C. After 30 min, 1.25 ml (about 20 mmol) of glacial acetic acid is added at room temperature and the solvent is evaporated at 40°C under reduced pressure. The residue is re-dissolved in 100 ml of water and loaded at the top of a column of 100 g of silanized Silica-gel (0.06-0.2 mm; Merck Co.) in water. The column is developed with 300 ml of water, then with 300 ml of a mixture acetonitrile:water 2:8 (v/v), with 300 ml of acetonitrile:water 1:1 (v/v), and finally with 1 L of a mixture acetonitrile:water adjusted to pH 3.0 with glacial acetic acid 1:1 (v/v), while collecting 200 ml fractions, which are monitored by HPLC.

Those fractions containing pure title compound are pooled and enough n-butanol is added to obtain, after concentration of the resulting mixture at 40°C under vacuum, a dry butanolic suspension of about 30 ml.

On adding 70 ml of ethyl ether, a solid separates which is collected by filtration, washed with 30 ml of ethyl ether and dried at room temperature in vacuo overnight to yield 1.9 g of the title compound, as the acetate.

Example 2

Preparation of Compound 2

To a stirred solution of 5 g (about 2.5 mmol) of a compound having formula I wherein $R^1$ is H and $R^2$ is $C_2H_5$ in 200 ml of methanol, 5 ml (about 66 mmol) of 40% (by weight) aqueous formaldehyde and 0.5 ml

(about 0.8 mmol) of anhydrous formic acid are added at room temperature, afterwards 5.4 g (about 146 mmol) of sodium borohydride is added portionwise, while mantaining the reaction mixture at 32-37 °C. After 20 min, 10 ml (about 160 mmol) of glacial acetic acid is added and the resulting solution is evaporated to dryness at 35 °C under reduced pressure. The residue is purified by reverse-phase column chromatography as described in Example 1, yielding 4.74 g (93%) of the title compound, as the acetate.

Compound 2 is also prepared with good yields (91%) following the above procedure but using a compound of formula I wherein $R^1$ is H and $R^2$ is $CH_3$ and acetaldehyde as the starting materials.

Example 3

Preparation of Compound 3

To a stirred suspension of 2 g (about 1 mmol) of teicoplanin $A_2$ complex in 100 ml of methanol, 4 ml (about 0.11 mol) of acetaldehyde and 0.6 ml (about 10 mmol) of glacial acetic acid are added, afterwards the reaction mixture is warmed to 30 °C and 0.74 g (about 20 mmol) of sodium borohydride are added portionwise, while maintaining the temperature between 35 °C and 40 °C. After min, 1.9 ml (about 30 mmol) of glacial acetic acid are added at room temperature and the resulting solution is worked up as described above, to yield 1.88 g (81%) of the title compound, as the acetate.

Example 4

Preparation of Compounds 4 and 5

To a stirred suspension of 2.5 g (about 2 mmol) of deglucoteicoplanin in 150 ml of methanol, 0.25 mmol of the proper aldehyde (formaldehyde or acetaldehyde) and 20 mmol of the corresponding carboxylic acid are added at room temperature. Then, 1.3 g (about 35 mmol) of sodium borohydride are added portionwise at 30-35 °C. After 30 min, the reaction mixture is adjusted to pH 3 with concentrated hydrochloric acid, and 300 ml of a mixture water:n-butanol 1:1 (v/v) is added under vigorous stirring. The organic layer is separated, washed with 100 ml of water, then it is concentrated to about 50 ml at 40 °C under reduced pressure. On adding 250 ml of ethyl ether, a solid separates which is collected by filtration and re-dissolved in 200 ml of a mixture water:acetonitrile 8:2 (v/v) adjusted to pH 3 with 1N hydrochloric acid. The resulting solution is loaded at the top of a column of 200 g of silanized Silica-gel (0.06-0.2 mm; Merck Co.) in water. The column is developed with 250 ml of water, then with 250 ml of a mixture water:acetonitrile 8:2 (v/v), with 500 ml of a mixture water:acetonitrile 1:1 (v/v) and finally with 750 ml of a mixture acetonitrile:0.05N hydrochloric acid 1:1 (v/v), while collecting 100 ml fractions. Those fractions containing pure (HPLC) title compounds are pooled and enough n-butanol is added to obtain, after concentration of the resulting mixture at 45 °C under vacuum, a dry butanolic solution (or suspension) of about 40 ml. Ethyl ether (160 ml) is added and the solid which precipitates is collected by filtration, washed with 100 ml of ethyl ether and dried at room temperature in vacuo overnight to give the title compounds, as the di-hydrochlorides.

Example 5

Preparation of Compound 6

To a stirred solution of 20 g (about 16 mmol) of a compound of formula I wherein A, B, M and $R^1$ are hydrogen atoms and $R^2$ is methyl, in 600 ml of methanol, 6 ml (about 170 mmol) of acetaldehyde and 1.5 ml (about 17 mmol) of glacial acetic acid are added. After 60 min, g (about 320 mmol) of sodium cyanoborohydride is added at 30 °C. The reaction mixture is then brought to pH 5 with glacial acetic acid, the solvent is evaporated at 45 °C under reduced pressure and the residue is chromatographed as previously described in Example 4, to give 19 g (89%) of the title compound, as the di-hydrochloride.

It is also possible to prepare the Compound 6 by starting from a compound of formula I wherein $R^1$ is H and $R^2$ is $C_2H_5$, according to the following procedure.

A stirred suspension of 2 g (about 1 mmol) of compound 1 in 30 ml of 0.5 M HCl solution in absolute 2,2,2-trifluoroethanol is heated at 80 °C for 12 h, while continuously bubbling anhydrous HCl. After cooling to room temperature, the reaction mixture is filtered and the collected solid is dissolved in 200 ml of $H_2O/CH_3CN$, 85/15. The resulting solution is loaded on a column of 200 g of silanized Silica-gel (0.06-0.2 mm, Merck Co.) which is then developed with a linear gradient from 10% to 50% of $CH_3CN$ in 0.001 N HCl in 10 hours at the rate of 200 ml/h, while collecting 10 ml fractions. Those containing pure (HPLC) title compound are pooled and solvents are evaporated at 40 °C under vacuum to give 1 g (about 98% yield) of compound 6, as the hydrochloride.

Example 6

By essentially following the procedure described in Example 1 but using teicoplanin $A_2$ factor 2 (2 g) as the teicoplanin starting material, Compound 7 as the acetate is obtained.

Example 7

By essentially following the procedure described in Example 2, but using teicoplanin $A_2$ factor 2 (2 g) as the teicoplanin starting material, Compound 8 as the acetate is obtained.

Example 8

By essentially following the procedure described in Example 3, but using teicoplanin $A_2$ factor 2 (2 g) as the teicoplanin starting material, Compound 9 as the acetate is obtained.

**Claims**

1. A reductive alkylation process for preparing $N^{15},N^{15}$-di-alkyl derivatives of teicoplanin compounds having the following formula:

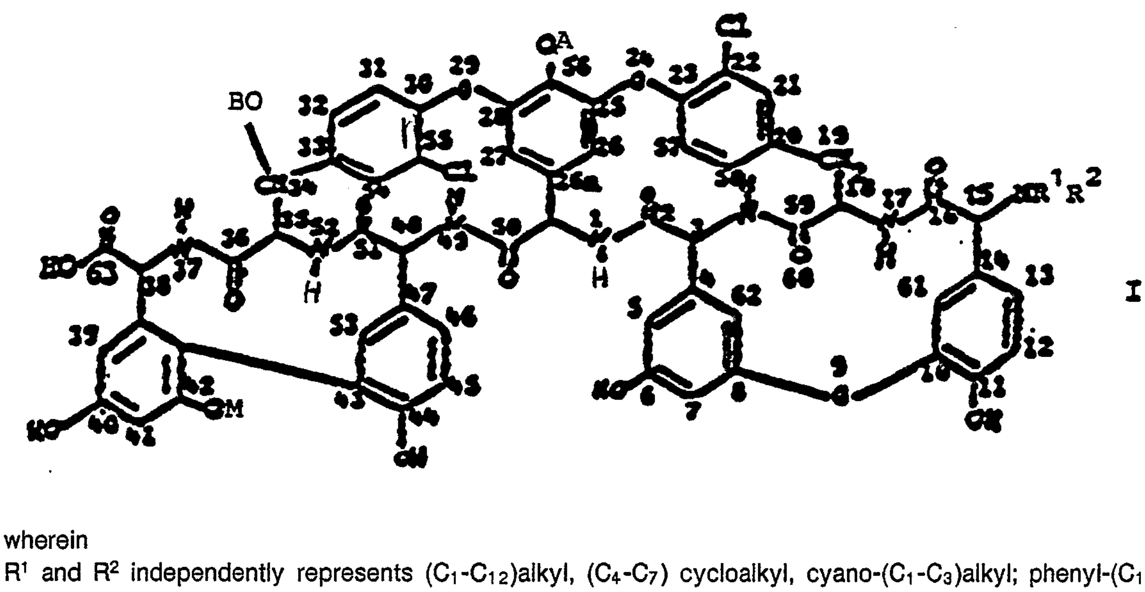

I

wherein

$R^1$ and $R^2$ independently represents $(C_1-C_{12})$alkyl, $(C_4-C_7)$ cycloalkyl, cyano-$(C_1-C_3)$alkyl; phenyl-$(C_1-C_4)$-alkyl wherein the phenyl group is optionally substituted in the position ortho, meta and/or para with 1 to 3 groups selected from $(C_1-C_4)$alkyl, nitro, halogen, $(C_1-C_4)$alkoxy, and phenyl;

A represents hydrogen or -N[$(C_9-C_{12})$aliphatic acyl]-beta-D-2-deoxy-2-amino-glucopyranosyl;

B represents hydrogen or N-acetyl-beta-D-2-deoxy-2-amino-glucopyranosyl;

M represents hydrogen or alpha-D-mannopyranosyl; with the proviso that B represents hydrogen only when A and M are simultaneously hydrogen; and the addition salts thereof,

characterized in that

a) to a suspension of a teicoplanin compound or a compound of formula II

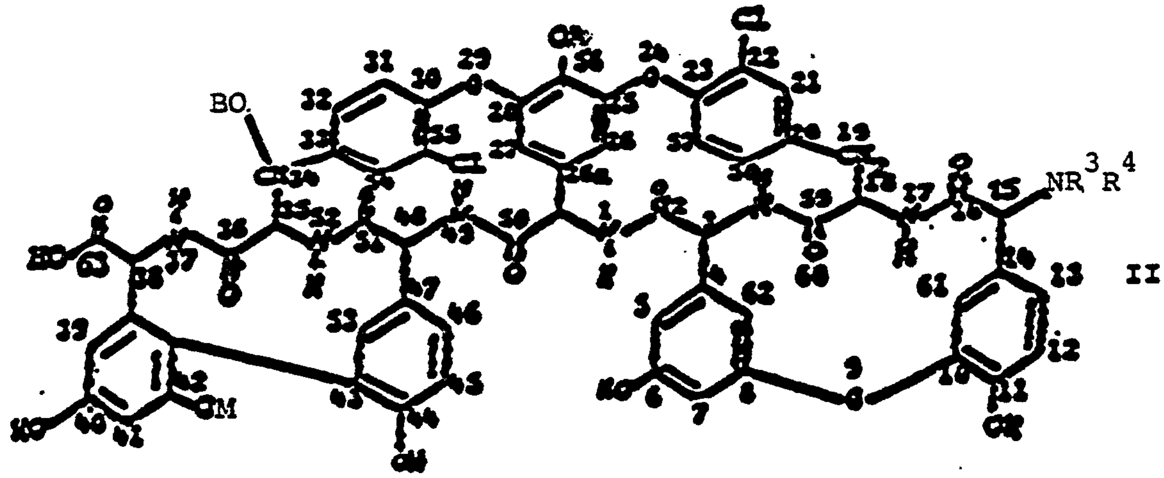

II

wherein $R^3$ is H and $R^4$ is H or $(C_1-C_{12})$alkyl, $(C_4-C_7)$cycloalkyl, cyano-$(C_1-C_3)$alkyl; phenyl $(C_1-C_4)$alkyl wherein the phenyl group is optionally substituted in the position ortho, meta and/or para with 1 to 3 groups selected from $(C_1-C_4)$alkyl, nitro, halogen, $(C_1-C_4)$alkoxy, and phenyl, in an organic polar protic solvent a molar excess of a carbonyl compound is added, said carbonyl compound having the same carbon skeleton of substituents $R^1$ and $R^2$ above and whose oxo group is on the carbon atom which is to link the $N^{15}$-amino group of teicoplanin moiety, said molar excess being of at least 30 times with respect to the teicoplanin starting material, in the presence of an organic acid of such strength and in such amount that a sample of the obtained mixture diluted with four volumes of water shows a pH between 2.5 and 4, said acid being further characterized in that, under the reaction conditions it cannot yield a reduction product which is a competitor of the carbonyl compound in the reaction with the $N^{15}$-amino group of teicoplanin moiety, and then

b) reacting the resulting mixture with an alkali metal borohydride at a temperature between 0 °C and 60 °C.

2. A process as in claim 1 wherein the carbonyl compound is an aldehyde and the organic acid is the corresponding carboxylic acid.

3. A process as in claim 1 wherein the carbonyl compound is a ketone and the organic acid is selected from a $(C_1-C_6)$ alkyl sulfonic acid and a $(C_6-C_{10})$aryl sulfonic acid.

4. A process as in claim 3 wherein the organic acid is selected from methanesulfonic acid and para toluene sulfonic acid.

5. A process as in claims 1, 2, 3 or 4 wherein the polar protic solvent is an alkanol having from 1 to 4 carbon atoms.

6. A process as in claim 5 wherein the alkanol is methanol.

7. A process as in claims 1, 2, 3, 4 or 5 wherein the temperature ranges from 30 °C to 40 °C.

8. A process as in claims 1, 2, 3, 4 or 5 wherein the molar excess of the carbonyl compound is from 30 to 300 times with respect to teicoplanin starting material.

9. A process as in claims 1, 2, 3, 4 or 5 further characterized in that the organic acid is in a molar excess from 5 to 20 times with respect to teicoplanin starting material.

10. A process as in claims 1, 2, 3, 4, 5 or 9 wherein the alkaline borohydrides are selected from sodium borohydride, potassium-cyano-borohydrides or sodium-cyano-borohydride.